# EUROPEAN PATENT APPLICATION

(11) **EP 4 718 216 A1**
(43) Date of publication of application: **01.04.2026**
(21) Application number: 25204482.1
(22) Date of filing: 25.09.2025
(51) Int. Cl.: G06F 3/01, G02B 21/36

(54) **APPARATUS FOR AN OPTICAL IMAGING SYSTEM, OPTICAL IMAGING SYSTEM, METHOD AND COMPUTER PROGRAM**

(30) Priority: 25.09.2024 DE 102024127703
(71) Applicant: Leica Instruments (Singapore) Pte Ltd., Singapore 618299 (SG)
(72) Inventor: THEMELIS, George, 618299 Singapore (SG)
(74) Representative: 2SPL Patentanwälte PartG mbB

(57) **Abstract**

Examples relate to an apparatus for an optical imaging system comprising one or more processors and one or more storage devices. The apparatus is configured to obtain sensor data indicative of a surrounding of the optical imaging system. Further, the apparatus may be configured to determine movement data indicative of a movement of a foot of a user of the optical imaging system. The movement data is determined based on the sensor data. Further, the apparatus is configured to generate adjustment data for adjusting a setting of the optical imaging system and to transmit the adjustment data for adjusting the setting of the optical imaging system.

## Description

### Technical field

Examples relate to an apparatus for an optical imaging system, an optical imaging system, a method, and a computer program.

### Background

Surgical microscopes are designed to reduce the disturbance to a surgeon to maintain focus on the operation. However, the surgical workflow is disturbed when the surgeon changes microscope parameters such as focus, zoom and field of view. Altering such microscope parameters requires the surgeon to free a hand to operate the microscope's interaction means as, for instance, microscope handles, buttons and adjustment wheels. Alternative, solutions such as a foot switch and a mouth switch which allow the surgeon to control the microscope with either foot or mouth may be used. However, such alternatives may be cost intensive. Therefore, optical imaging system mostly rely on hand control, which may disturb an operation and thus decrease a user experience. Thus, there may be a desire for an improved concept for controlling an optical imaging system, e.g., a setting of an optical imaging system.

### Summary

This desire is addressed by the subject-matter of the independent claims.

The concept proposed in the present disclosure is based on the insight, that a concept for controlling of an optical imaging system, in particular a setting of the optical imaging system, can be improved by using a virtual control menu, e.g., a virtual foot switch. The virtual control menu may be used to replace a physical control, such like a physical foot switch, thus reducing costs and/or complexity of the hardware of the optical imaging system. The virtual control menu may allow the user to adjust the setting of the optical imaging system, e.g., a setting of a microscope part of the optical imaging system. Further, the virtual control menu may improve a user experience, since an arrangement of a physical component can be avoided. That is, the usability by the user of the virtual control menu may be better than this of a physical component, such like a foot switch.

Examples provide an apparatus for an optical imaging system comprising one or more processors and one or more storage devices. The apparatus is configured to obtain sensor data indicative of a surrounding of the optical imaging system. The surrounding of the optical imaging system may be an environment in which the optical imaging system is placed. Further, the apparatus may be configured to determine movement data indicative of a movement of a foot of a user of the optical imaging system. The movement data is determined based on the sensor data. The movement data may, for example, indicate a gesture performed by the foot of the user. Further, the apparatus is configured to generate adjustment data for adjusting a setting of the optical imaging system and to transmit the adjustment data for adjusting the setting of the optical imaging system. The adjustment data is generated based on the movement data. Using the movement data may allow a user of the optical imaging system to control the optical imaging system with the foot without a physical control, such like a foot switch. That is, the movement data can be used to set the setting of the optical imaging system using a virtual control menu. In this way, the user can control the setting of the optical imaging system without a need to perform certain measure with the hands and without additional physical hardware.

In an example, the apparatus may be configured to obtain trigger data indicative of a trigger event for generating the adjustment data and determine the adjustment data based on the trigger data. The trigger event may allow to avoid an unintended usage of the virtual control menu, e.g., an unintended initialization of the virtual control menu. In this way, an unintended adjustment of the setting of the optical imaging system can be avoided. Thus, a usability of the virtual control menu can be improved.

In an example, the trigger event may be a voice utterance of the user and/or a predefined movement of the foot. For example, the virtual control menu can be initialized based on a user speech or a predefined movement of the foot, such like a predefined gesture. For example, adjustment of a setting of the optical imaging system, which may be safety relevant for a surgery, may for safety reason only be performed when the movement data and the trigger data indicate an intention of the user to adjust the setting.

In an example, the apparatus may be configured to generate, in answer to obtaining the trigger data, activation data for informing the user about an activation of a function for adjusting the setting of the optical imaging system with the foot and to transmit the activation data for informing the user about the activation. Informing the user about the activation may allow the user to take certain counter measures when the activation was unintended. Further, informing the user about the activation may allow the user to more precisely use the function for adjusting the setting. The function for adjusting the setting may be a virtual control menu, a virtual foot switch or a subfunction of a virtual foot switch.

In an example, the apparatus may be configured to generate, in answer to generating the adjustment data, feedback data for providing the user feedback about the determined adjustment data and transmit the feedback data for providing the user the feedback about the control of the optical imaging system. The feedback data may allow the user to check the adjustment of the setting which could be caused by the adjustment data. For example, the feedback data may indicate that the apparatus will control the optical imaging system to adjust a certain setting. When the user is not satisfied with the control indicated by the adjustment data, the user may cancel the adjustment. In this way, an unintended adjustment of the setting (e.g., due to a wrong determination of a gesture performed by the user) can be avoided. Optionally or alternatively, the user can be informed about a setting that has been made in order to draw their attention to the setting that has been made.

In an example, the feedback data may comprise a control signal for controlling an ultrasonic device for informing the user about the adjustment data. Thus, the user can receive physical feedback. For example, the user may receive physical feedback when a certain functionality of the function was activated, e.g., the apparatus has determined that the user intended to press a button of the virtual control menu.

In an example, the feedback data may comprise a visual representation for informing the user about the adjustment data. The apparatus may be configured to obtain sample data of an optical imaging sensor indicative of an image of a sample and to generate a composite image comprising the image of the sample and the visual representation. Further, the apparatus may be configured to transmit the composite image for displaying on a display device. For example, the user can be informed about the function for adjusting the setting, e.g., the visual representation may comprise a virtual control menu. Displaying the virtual control menu and the image of the sample may facilitate a control of the optical imaging by the user without interrupting a workflow. Thus, a user experience can be improved.

In an example, the visual representation may indicate the movement of the foot of the user in real time. In this way, the user can receive feedback about possible control movements, e.g., which movement of the foot could trigger which functionality and/or adjustment of the optical imaging system. For example, if the virtual control menu comprises different buttons for adjusting the setting, a control can be facilitated by displaying the user the current foot position with respect to the buttons, which could be selected/pressed by foot.

In an example, the apparatus may be configured to initialize the function for adjusting the setting of the optical imaging system, such that the foot of the user is positioned at a default position for using the function for adjusting the setting of the optical imaging system. Positioning the foot of the user at a default position may allow to provide an intuitive control of function for adjusting the setting. For example, the user can perform the same movement of the foot regardless of the position of the user relative to a physical control. In this way, a userfriendly adjustment can be made.

In an example, the apparatus may be configured to generate the adjustment data by determining, based on the movement data, gesture data indicative of a gesture performed by the user to control the optical imaging system with the foot. In this way, the user may control an adjustment of the setting by performing a gesture with the foot.

In an example, the apparatus may be configured to determine, based on the movement data, an authorization of the user of the function for adjusting the setting of the optical imaging system and to adjust a functionality of the function for adjusting the setting of the optical imaging system based on the determined authorization. For example, different user may have different authorizations to adjust the setting of the optical imaging system, e.g., a main surgeon may have less restrictions for adjustment than an assistant. In this way, the control of the setting can be individualized. Further, individualizing the control may prevent misusage by an unskilled user.

Examples provide an optical imaging system comprising an apparatus as described above.

In an example, the optical imaging system may further comprise an illumination source configured to illuminate a part of the surrounding of the optical imaging system with non-visible light. In this way, a distraction of the user and/or an impairment of the image of the sample acquired with the microscope can be reduced or even avoided.

Examples provide a method for an optical imaging system. The method comprises obtaining sensor data indicative of a surrounding of the optical imaging system and determining, based on the sensor data, movement data indicative of a movement of a foot of a user of the optical imaging system. Further, the method comprises generating, based on the movement data, adjustment data for adjusting a setting of the optical imaging system and transmitting the adjustment data for adjusting the setting of the optical imaging system.

Various examples of the present disclosure relate to a corresponding computer program with a program code for performing the above method when the computer program is executed on a processor.

### Short description of the Figures

Some examples of apparatuses and/or methods will be described in the following by way of example only, and with reference to the accompanying figures, in which
Figs. 1a and 1b show schematic diagrams of examples of a system for an optical imaging system and of a corresponding optical imaging system;
Fig. 2 shows a schematic view of a virtual control system utilizing a movement of a foot;
Fig. 3 shows a flow chart of an example of a method for an optical imaging system; and
Fig. 4 shows a schematic diagram of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

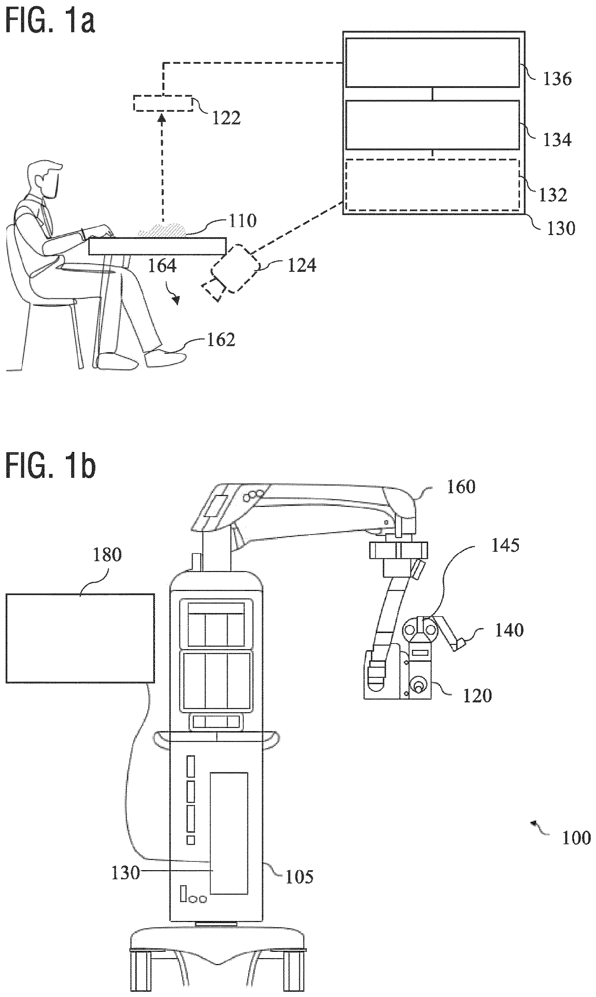

Figs. 1a and 1b show schematic diagrams of an example of an apparatus 130 for an optical imaging system 100 and of a corresponding optical imaging system 100 comprising the apparatus 130. The apparatus 130 is tasked with controlling various aspects of a microscope 120 of the optical imaging system 100, which may be a surgical optical imaging system, and of the entire optical imaging system 100 and/or with processing various types of sensor data of the optical imaging system 100. Consequently, the apparatus 130 may be implemented as a computer system, which interfaces with the various components of the optical imaging system 100, e.g., the optical imaging sensor 122. The apparatus 130 may be part of the optical imaging system 100. Alternatively, the apparatus 130 may be communicatively coupled to the optical imaging system 100. For example, the apparatus 130 may be a ready-to-use-module, which could be connected to the optical imaging system 100.

The apparatus 130 comprises, as shown in Fig. 1a, one or more processors 134 and one or more storage devices 136. Optionally, the apparatus 130 further comprises one or more interfaces 132. The one or more processors 134 are coupled to the one or more storage devices 136 and to the optional one or more interfaces 132. In general, the functionality of the apparatus 130 may be provided by the one or more processors 134 (for generating adjustment data), in conjunction with the one or more interfaces 132 (for exchanging information, e.g., with the optical imaging sensor 122, to transmit the adjustment data) and/or with the one or more storage devices 136 (for storing and/or retrieving information).

The apparatus 130 is configured to obtain sensor data indicative of a surrounding of the optical imaging system 100. The sensor data may be obtained by receiving from an environmental sensor 124, such like a camera, an infrared sensor. Alternatively, the sensor data may be obtained by measuring. For example, the apparatus 130 may comprise an environmental sensor 124 and thus may control the environmental sensor 124 to acquire the sensor data.

The surrounding of the optical imaging system 100 may be an actual physical environment in which the optical imaging system 100 is placed, such like a laboratory, an operating room. That is, the environmental data may indicate a tangible, real-world setting or space comprising the optical imaging system 100. Optionally or alternatively, the environmental data may comprise or may be restricted to a user interaction area. The user interaction area may be a space around the optical imaging system 100 that the user interacts with, such as the stage where the slides are placed, adjustment knobs for focusing, an area for manipulating or changing the objective lenses, a (predefined) area for detecting a movement of the foot of the user, e.g., a foot space 164. The user interaction area may allow to improve and/or facilitate obtaining the sensor data, e.g., a camera can be directed only to a foot space 164 formed by the optical imaging system 100 and/or another structure associated with the optical imaging system 100, e.g., an operating table on which the sample 110 is placed.

Further, the apparatus 130 may be configured to determine movement data indicative of a movement of a foot 162 of a user of the optical imaging system 100. The movement data is determined based on the sensor data. That is, the apparatus 130 may post process the sensor data to obtain the movement data. The movement of the foot 162 can be an actual movement. The movement data may, for example, indicate a gesture performed by the foot of the user. Alternatively, no movement of the foot 162 can be determined based on the sensor data. That is, also the determination of no movement of the foot 162 can be used to generate adjustment data, e.g., when trigger data was obtained, or when no movement is associated with a setting of the optical imaging system 100, e.g., a default setting.

Further, the apparatus 130 is configured to generate adjustment data for adjusting a setting of the optical imaging system 100 based on the movement data. A setting of the optical imaging system may refer to a setup of the optical imaging system 100. The setting may define a current mode, such like white light imaging, a magnification, a field of view, a tuning of a condenser lens or other parameters of the optical imaging system 100. For example, the setting of the optical imaging system 100 may affect the image acquisition of the optical imaging system 100. Thus, adjusting the setting of the optical imaging system 100 may allow to adjust the image acquisition. Thus, a user can adjust an image displayed on a display device 180 with a movement of the foot 162, for example.

Further, the apparatus 130 is configured to transmit the adjustment data for adjusting the setting of the optical imaging system 100. This may allow to adjust the setting of the optical imaging system 100 without using a hand. Further, a traditional physical foot switch can be replaced using the apparatus 130. A traditional physical foot switch may come with several disadvantages. For example, it can be cumbersome and can require physical contact, leading to hygiene issues. Further, it can be limited in the number of functions it can control without increasing complexity. Also, it can be prone to wear and tear, leading to potential malfunctions and the user may need to shift their focus to locate and operate the physical foot switch, disrupting their workflow. It is a finding of the inventors, that a physical hardware, such like a physical foot switch, can be replaced by the apparatus 130, e.g., with a virtual control menu generated by the apparatus 130. This can be achieved by using environmental data, e.g., obtained by a camera-based system, which acquires data indicating a movement of a foot 162. For example, the apparatus 130 can determine a gesture performed by the user based on the environmental data. That is, the apparatus 130 may process the environmental data for adjusting a setting of the optical imaging system 100, e.g., by providing a function for adjusting the setting of the optical imaging system 100. In this way, a need for physical contact can be eliminated and/or an operational efficiency can be enhanced.

For example, the apparatus 130 can be used in conjunction with an environmental sensor 124 such as a camera-based system. The camera-based system can measure environmental data indicating a movement of a foot 162. The apparats 130 may process the environmental data for controlling the setting of the optical imaging system 100. In this way, the apparatus 130 can provide hygienic, efficient, low-cost and/or customizable control for a user, e.g., a surgeon.

Thus, the apparatus 130 may reduce the need for physical contact, enhancing hygiene and reducing infection risk, may allow for more intuitive and flexible control of the optical imaging system 100, improving efficiency, may reduce disruptions in the workflow as a user does not need to locate and operate a physical hardware and/or does not take up space in the operating room, freeing up valuable space for other equipment and personnel.

The proposed concept may be built around two main components - the microscope 120, which comprises the optical components used to view the sample 110, and the apparatus 130, which may be used to control the optical imaging system 100, process sensor data of the microscope 120, e.g., the optical imaging sensor 122, and/or to generate adjustment data.

In general, a microscope, such as the microscope 120, is an optical instrument that is suitable for examining objects that are too small to be examined by the human eye (alone). For example, a microscope 120 may provide an optical magnification of a sample, such as a sample 110 shown in Fig. 1a. In modern microscopes, the optical magnification is often provided for a camera or an imaging sensor, such as the optical imaging sensor 122 of the microscope 120. The microscope 120 may further comprise one or more optical magnification components that are used to magnify a view of the sample 110, such as an objective.

There are a variety of different types of optical imaging systems. If the optical imaging system 100 is used in the medical or biological fields, the sample 110 may be a sample of organic tissue, e.g., arranged within a petri dish or present in a part of a body of a patient. In some examples of the present disclosure, e.g., as shown in Fig. 1b, the optical imaging system 100 may be a surgical optical imaging system, e.g., an optical imaging system that is to be used during a surgical procedure, such as an oncological surgical procedure or during tumor surgery. However, the proposed concept may also be applied to other types of microscopes, e.g., a microscope in a laboratory or a microscope for the purpose of material inspection.

Fig. 1b shows a schematic diagram of an example of a surgical optical imaging system 100 comprising the microscope 120 and the apparatus 130. In general, a (surgical) optical imaging system is a system that comprises a microscope 120 and additional components, which are operated together with the microscope 120. In other words, a (surgical) optical imaging system is a system that comprises the microscope 120 and one or more additional components, such as the apparatus 130 (which may be a computer system being adapted to control the microscope 120 and, for example, generate the adjustment data), an illumination system (which is used to illuminate a sample being imaged by the microscope 120 and can be controlled by the apparatus 130), additional sensors, displays etc.

The optical imaging system 100 shown in Fig. 1b comprises a number of optional components, such as a base unit 105 (which may comprise the apparatus 130) with a stand, ocular displays 140, 145 that are arranged at the microscope 120, a display device 180, and a (robotic or manual) arm 160 which holds the microscope 120 in place, and which is coupled to the base unit 105 and to the microscope 120. In general, these optional and non-optional components may be coupled to the apparatus 130, which may be configured to control and/or interact with the respective components.

In an example, the apparatus 130 may be configured to obtain trigger data indicative of a trigger event for generating the adjustment data and determine the adjustment data based on the trigger data. The trigger data may allow to determine an intention of the user to adjust a setting of the optical imaging system 100. For example, the user may want to use their foot 162 to adjust the setting of the optical imaging system 100. Optionally, a virtual control menu may be used to facilitate the adjustment of the optical imaging system 100. The virtual control menu, such like a virtual foot switch, may be displayed on a display device 180. For example, the virtual control menu may be overlaid with an image of the sample 110 to be displayed on the same display device 180.

In an example, the trigger event may be a voice utterance of the user and/or a predefined movement of the foot 162. The trigger event may indicate a usage intention of the user, such that the function for adjusting the setting can be initialized based on the trigger event. For example, the user may use a voice command for initializing the function for adjusting the setting. The apparatus 130 may process the trigger data to determine the voice utterance and/or the predefined movement of the foot 162 assigned to initializing the function for adjusting the setting. For example, the user utterance may be "start control menu" and the apparatus 130 may initialize the function for adjusting the setting based on this user utterance. In this way, the usability of the function for adjusting the setting can be improved.

Further, the trigger data can be used to improve a reliability of the function for adjusting the setting. For example, specific adjustment of the setting of the optical imaging system 100, which may have a safety relevance on the usability of the optical imaging system 100 during a surgery, may be only allowed when the trigger data and the movement data indicate the same intention of the user. That is, the trigger data can be used as fallback option for adjusting the setting of the optical imaging system 100. For example, a magnification, a depth of focus, a light intensity may only be adjusted when the movement data and the trigger data indicate the same intention of the user.

In an example, the apparatus 130 may be configured to generate, in answer to obtaining the trigger data, activation data for informing the user about an activation of a function for adjusting the setting of the optical imaging system 100 with the foot 162 and to transmit the activation data for informing the user about the activation. The function for adjusting the setting may be a virtual control menu, a virtual foot switch or a subfunction of a virtual foot switch. A virtual control menu can be a software-based interface that allows users to control and adjust various settings of the optical imaging system. Instead of physical knobs or buttons, the virtual control menu presents on-screen options (e.g., displayed on the display device 180) that the user can interact with, e.g., using movements of the foot 162 and optionally other commands such like voice commands. A virtual control menu might allow the user to adjust magnification, focus, lighting, or camera setting of the optical imaging system, for example. The virtual control menu may be more versatile than physical hardware, as it can offer more detailed settings and customization options within a single, easily accessible display device 180. For example, the virtual control menu may be a representation of a virtual foot switch. The virtual foot switch can be used to replace (and optionally enhance) the functionality of a physical foot switch. That is, the function for adjusting the setting of the optical imaging system 100 can replace a physical hardware of the optical imaging system 100.

The activation data may allow to inform the user by acoustic feedback, ultrasonic feedback and/or visual feedback about the activation of the function for adjusting the setting. For example, the user may receive ultrasonic feedback when the function for adjusting the setting has been activated.

In an example, the apparatus 130 may be configured to generate, in answer to generating the adjustment data, feedback data for providing the user feedback about the determined adjustment data and transmit the feedback data for providing the user the feedback about the control of the optical imaging system 100. The feedback data may allow to inform the user about a planned adjustment of the setting of the optical imaging system 100. That is, the feedback data ay improve a reliability of the adjustment of the setting of the optical imaging system 100, since a false-positive event can be avoided. For example, when the user does not want an adjustment of the setting according to the feedback data, the user may cancel the planned adjustment. The feedback data may be displayed on a display device 180, e.g., overlaid with an image of the sample 110. Thus, the feedback data may allow the user to check the planned adjustment of the optical imaging system 100.

In an example, the feedback data may comprise a control signal for controlling an ultrasonic device for informing the user about the adjustment data. That is, the apparatus 130 can control or trigger an ultrasonic device for providing ultrasonic feedback to the user. Ultrasonic feedback can be used to confirm when a movement such like a predefined gesture has been recognized by the apparatus 130. For example, when a surgeon swipes to change magnification or moves the foot 162 to press a button of a virtual foot switch (displayed on a display device 180) the user might feel a brief pulse or vibration in the air, confirming the intended action, e.g., confirming pressing the virtual button. In this way, the user can receive feedback comparable to a physical hardware, which may improve a usability and/or user experience.

In an example, the feedback data may comprise a visual representation for informing the user about the adjustment data. The apparatus 130 may be configured to obtain sample data of an optical imaging sensor 122 indicative of an image of a sample 110 and to generate a composite image comprising the image of the sample 110 and the visual representation. Further, the apparatus 130 may be configured to transmit the composite image for displaying on a display device 180. The visual representation may comprise a user interface showing a virtual control menu, e.g., a virtual foot switch. That is, the user can view a virtual representation of a foot switch, such that the user can adjust the setting of the optical imaging system using a virtual foot switch. For example, the visual representation can display a button press of the virtual foot switch. Optionally or alternatively, the visual representation may show information about a planned or performed adjustment of the setting. For example, the visual representation may show that the setting of the optical imaging system 100 is to be adjusted or was adjusted.

In an example, the visual representation may indicate the movement of the foot 162 of the user in real time. For example, a virtual control menu may be displayed on a display device 180 and could be updated in real-time as movement of the foot 162 such like gestures are recognized, allowing the surgeon to see and select options without physically interacting with the optical imaging system 100. Providing real time feedback may improve the usability of the function for adjusting the setting of the optical imaging system 100.

In an example, the apparatus 130 may be configured to initialize the function for adjusting the setting of the optical imaging system, such that the foot 162 of the user is positioned at a default position for using the function for adjusting the setting of the optical imaging system 100. The default position may be a center position of the virtual control menu, e.g., a center position of a virtual foot switch, a position at a corner, a position at a side, for example. Positioning the foot 162 at the default position may allow the user to perform certain movements with the foot 162 in a predefined way independently of the current position of the foot 162. For example, a virtual foot switch may comprise buttons at corners and thus by positioning the foot 162 at a center the user can perform a known movement of the foot 162 to use the button, e.g., to trigger an action correlated with the button. That is, a movement of the foot may be a movement along a predefined way, which could be performed using the default position.

In an example, the apparatus 130 may be configured to generate the adjustment data by determining, based on the movement data, gesture data indicative of a gesture performed by the user to control the optical imaging system with the foot 162. In this way, the user may control an adjustment of the setting by performing a gesture with the foot 162. The apparatus 130 may compare the movement of the foot 162 with predefined gestures. The predefined gestures may be retrieved from a storage device, e.g., the storage device 136. That is, the apparatus 130 can detect whether the user has performed a gesture for adjusting the setting of the optical imaging system 100 or not.

The predefined gestures retrieved from a storage device may be personalized. That is, the apparatus 130 may determine an identity of the user and may retrieve gestures assigned to the identified user. In this way, each user can use their own gestures. Thus, customizable gesture recognition settings for process control can be provided.

The movement data may indicate a movement of the foot 162 of the user, i.e., a general action that involve shifting or positioning the foot 162, like moving the foot from side to side or raising it up and down. The movement of the foot 162 can be simple and optionally might not have a specific purpose beyond the physical act itself. That is, the movement data may comprise each kind of movement of the foot 162 of the user. The apparatus 130 may determine a gesture performed by the user based on the movement of the foot 162. A gesture, on the other hand, may be a deliberate movement with a specific intent or meaning. A gesture is often used to control or interact with a device or system, e.g., the optical imaging system 100. For example, a swipe gesture might be used to scroll through a virtual control menu, while a pinch gesture might be used to zoom in on a display device 180.

However, a movement can indicate intent or provide information, even if it is not as deliberate or specific as gestures. For example, no movement, i.e., the absence of movement, could signal that everything is fine or that no further action is needed. For instance, in a gesture-controlled system, if the user holds their foot 162 still, it might indicate that they want to maintain the current setting or that they are finished with their current interaction. For example, minimal movement, i.e., small or subtle movements could be used to indicate fine-tuning or making minor adjustments, implying that the user is making precise changes rather than performing a major action. Thus, while gestures are typically defined as intentional and specific movements for controlling devices, general movements (or the lack thereof) can also convey information or intentions in various contexts. Therefore, the movement indicated by the movement data can be processed by the apparatus 130 to determine the intention of the user to adjust the setting of the optical imaging system 100. The intention can be determined based on gesture detection and/or a movement of the foot (which is no gesture). For example, an initialization of the function for adjusting the setting may be triggered by a user utterance, e.g., "start control menu" in conjunction with a specific movement of the foot 162. For example, the function for adjusting the setting may only be initialized when the movement data indicates no movement of the foot 162 of the user during obtaining the trigger data or the trigger event. In this way, the movement of the foot 162 and the user utterance can be combined to improve usage of the function for adjusting the setting, e.g., an initialization of the function for adjusting the setting.

In an example, the apparatus 130 may be configured to determine, based on the movement data, an authorization of the user of the function for adjusting the setting of the optical imaging system 100 and to adjust a functionality of the function for adjusting the setting of the optical imaging system 100 based on the determined authorization. For example, different user may have different rights to adjust the setting of the optical imaging system 100. For example, a user, e.g., a main surgeon, may wear an indication element on his foot 162. The indication element may be a QR sticker or a marker-made sign on the shoe that could be used to identify the user, for example. Thus, the movement data may indicate the identity of the user. For example, a main surgeon may have the authorization to adjust more settings of the optical imaging system 100 than an assistant. Thus, the apparatus 130 may adjust the functionality of the function for adjusting the setting accordingly. For example, the movement data may indicate a movement of foot of another user, such as an assistant, and the identity of the second user (e.g., again using an indication element). Therefore, the apparatus 130 can determine a movement performed by the other user and may adjust the functionality of the function for adjusting the setting accordingly. In this way, individualization of the adjustment of the optical imaging system can be achieved. Further, a misusage by an unskilled user performing a (unintended) movement with the foot which could cause an adjustment of the setting could be prevented.

In an example, the optical imaging system 100 may further comprise an illumination source configured to illuminate a part of the surrounding of the optical imaging system with nonvisible light. In this way, a distraction of the user and/or an impairment of the image of the sample acquired with the microscope can be reduced or even avoided. For example, the illumination source may be part of the environmental sensor 124. The environmental sensor 124 and/or the illumination source may be nonvisible wavelengths to avoid destruction and/or light contamination of the operating room (for example, the operating room needs to be darkened for fluorescence imaging).

As shown in Fig. 1a the optional one or more interfaces 132 is coupled to the respective one or more processors 134 at the apparatus 130. In examples the one or more processors 134 may be implemented using one or more processing units, one or more processing devices, any means for processing, such as a processor, a computer or a programmable hardware component being operable with accordingly adapted software. Similar, the described functions of the one or more processors 134 may as well be implemented in software, which is then executed on one or more programmable hardware components. Such hardware components may comprise a general-purpose processor, a Digital Signal Processor (DSP), a micro-controller, etc. The one or more processors 134 is capable of controlling the one or more interfaces 132, so that any data transfer that occurs over the one or more interfaces 132 and/or any interaction in which the one or more interfaces 132 may be involved may be controlled by the one or more processors 134.

In an embodiment the apparatus 130 may comprise a memory, e.g., the one or more storage devices 136 and at least one or more processors 134 operably coupled to the memory and configured to perform the method described below.

In examples the one or more interfaces 132 may correspond to any means for obtaining, receiving, transmitting or providing analog or digital signals or information, e.g. any connector, contact, pin, register, input port, output port, conductor, lane, etc. which allows providing or obtaining a signal or information. The one or more interfaces 132 may be wireless or wireline and it may be configured to communicate, e.g., transmit or receive signals, information with further internal or external components.

The apparatus 130 may be a computer, processor, control unit, (field) programmable logic array ((F)PLA), (field) programmable gate array ((F)PGA), graphics processor unit (GPU), application-specific integrated circuit (ASICs), integrated circuits (IC) or system-on-a-chip (SoCs) system.

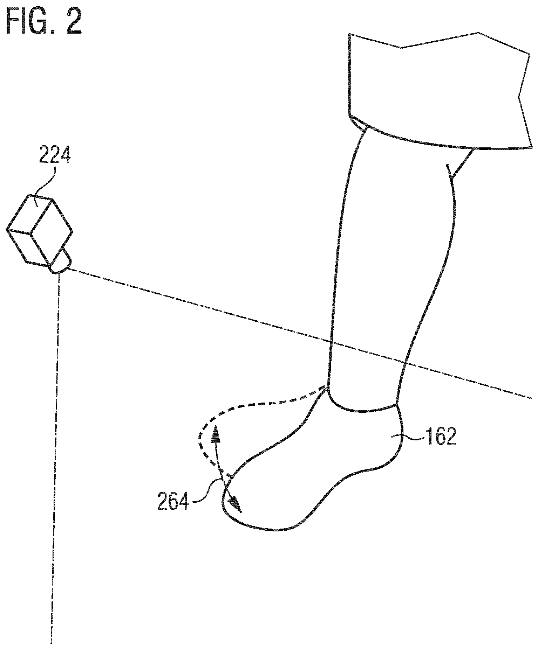

More details and aspects are mentioned in connection with the examples described below. The example shown in Fig. 1 may comprise one or more optional or additional features corresponding to one or more aspects mentioned in connection with the proposed concept or one or more examples described below (e.g., Fig. 2 - 4).

Fig. 2 shows a schematic view of a virtual control system utilizing a movement 264 of a foot 162. The virtual control system may comprise an environmental sensor 224. The environmental sensor 224 can be an infrared sensor or a motion camera (e.g., a high-resolution camera), for example. The environmental sensor 224 may be configured to detect a movement 264 of the foot 162 of the user. For example, the environmental sensor 224 may be configured to acquire an image of the surrounding of the optical imaging system, especially of a foot space. The environmental sensor 224 can be mounted on a surgical table, a chair of the surgeon, or a standalone stand, for example.

The sensor data indicative of a surrounding of the optical imaging system may be transmitted from the environmental sensor 224 to an apparatus part of the virtual control system, e.g., the apparatus as described with reference to Fig. 1. The apparatus may process the sensor data to determine or recognize a foot gesture and/or movement 264 of the foot 162. When the sensor data is processed by the environmental sensor 224, the environmental sensor 224 may be part of the apparatus. Optionally, the apparatus may determine an authorization of the user. For example, the user may wear an indication element. For example, a shoe, sock, foot recognition can be used to configure and/or customize the adjustment of the setting of the optical imaging system. For example, the apparatus may obtain information to recognize the shoe or sock of the user.

The apparatus may translate the determined or recognized gesture and/or movement 264 of the foot 162 into a command for the optical imaging system. The command may be part of the adjustment data. Thus, the command can be used for adjusting the setting of the optical imaging system.

The virtual control system shown in Fig. 2 illustrates a system where a camera detects a movement 264 of the foot 162 and/or a gesture. The camera may be mounted to provide a clear view of the foot 162, allowing for movement-based control commands, enhancing hygiene, and/or freeing up space in the operating room. The virtual control system, e.g., a virtual footswitch system, may provide a significant advancement over traditional foot switches, offering a more hygienic, efficient, space-saving, and/or customizable solution for adjusting a setting of the optical imaging system.

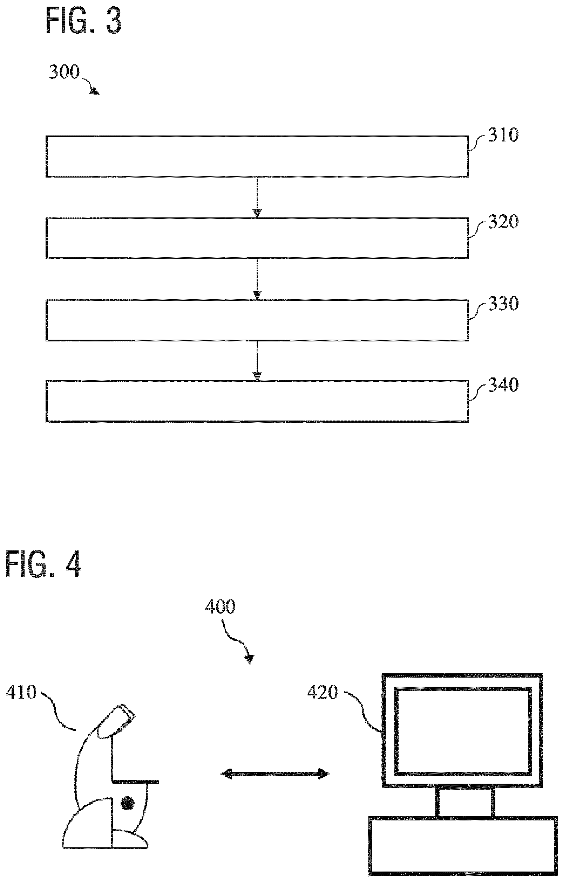

More details and aspects are mentioned in connection with the examples described above and/or below. The example shown in Fig. 2 may comprise one or more optional or additional features corresponding to one or more aspects mentioned in connection with the proposed concept or one or more examples described above (e.g., Fig. 1) and/or below (e.g., Fig. 3 -4).

Fig. 3 shows a flow chart of an example of a method 300 for an optical imaging system. The method 300 comprises obtaining 310 sensor data indicative of a surrounding of the optical imaging system and determining 320, based on the sensor data, movement data indicative of a movement of a foot of a user of the optical imaging system. Further, the method 300 comprises generating 330, based on the movement data, adjustment data for adjusting a setting of the optical imaging system and transmitting 340 the adjustment data for adjusting the setting of the optical imaging system.

More details and aspects are mentioned in connection with the examples described above and/or below. The example shown in Fig. 3 may comprise one or more optional or additional features corresponding to one or more aspects mentioned in connection with the proposed concept or one or more examples described above (e.g., Fig. 1 - 2) and/or below (e.g., Fig. 4).

Some embodiments relate to a microscope comprising an apparatus as described in connection with Fig. 1. Alternatively, a microscope or an optical imaging system can be communicatively connected to an apparatus as described in connection with Fig. 1. Fig. 4 shows a schematic illustration of a system 400, e.g., an optical imaging system, configured to perform a method described herein, e.g., with reference to Fig. 3. The system 400 comprises a microscope 410 and a computer system 420. The microscope may comprise the apparatus as described above, e.g., with reference to Fig. 1. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g., camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

More details and aspects are mentioned in connection with the examples described above. The example shown in Fig. 4 may comprise one or more optional or additional features corresponding to one or more aspects mentioned in connection with the proposed concept or one or more examples described above (e.g., Fig. 1 - 3).

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a nontransitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

If some aspects have been described in relation to a device or system, these aspects should also be understood as a description of the corresponding method and vice versa. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

The aspects and features described in relation to a particular one of the previous examples may also be combined with one or more of the further examples to replace an identical or similar feature of that further example or to additionally introduce the features into the further example.

### List of reference Signs

100 optical imaging system
105 base
110 sample
120 microscope
122 optical imaging sensor
124 environmental sensor
130 apparatus
132 interface
134 processor
136 storage device
140, 145 ocular display
160 arm
162 foot
164 foot space
180 display device
224 environmental sensor
264 movement
300 method for an optical imaging system
310 obtain sensor data
320 determine movement data
330 generate adjustment data
340 transmit the adjustment data
400 system
410 microscope
420 computer system

## Claims

1. An apparatus (130) for an optical imaging system (100), comprising one or more processors (134) and one or more storage devices (136), wherein the apparatus (130) is configured to:
obtain sensor data indicative of a surrounding of the optical imaging system (100);
determine, based on the sensor data, movement data indicative of a movement of a foot (162) of a user of the optical imaging system (100);
generate, based on the movement data, adjustment data for adjusting a setting of the optical imaging system (100); and
transmit the adjustment data for adjusting the setting of the optical imaging system (100).

2. The apparatus (130) according to claim 1, wherein the apparatus (130) is configured to
obtain trigger data indicative of a trigger event for generating the adjustment data; and
determine the adjustment data based on the trigger data.

3. The apparatus (130) according to claim 2, wherein
the trigger event is at least one of a voice utterance of the user and a predefined movement of the foot (162).

4. The apparatus (130) according to any one of the claims 2 or 3, wherein the apparatus (130) is configured to:
generate, in answer to obtaining the trigger data, activation data for informing the user about an activation of a function for adjusting the setting of the optical imaging system (100) with the foot (162); and
transmit the activation data for informing the user about the activation.

5. The apparatus (130) according to any one of the preceding claims, wherein the apparatus (130) is configured to:
generate, in answer to generating the adjustment data, feedback data for providing the user feedback about the determined adjustment data; and
transmit the feedback data for providing the user the feedback about the control of the optical imaging system (100).

6. The apparatus (130) according to claim 5, wherein
the feedback data comprises a control signal for controlling an ultrasonic device for informing the user about the adjustment data.

7. The apparatus (130) according to claim 5 or 6, wherein
the feedback data comprises a visual representation for informing the user about the adjustment data; and wherein the apparatus (130) is configured to:
obtain sample data of an optical imaging sensor indicative of an image of a sample (110);
generate a composite image comprising the image of the sample (110) and the visual representation; and
transmit the composite image for displaying on a display device.

8. The apparatus (130) according to claim 7, wherein
the visual representation indicates the movement of the foot (162) of the user in real time.

9. The apparatus (130) according to any one of the claims 2-8, wherein the apparatus (130) is configured to
initialize the function for adjusting the setting of the optical imaging system (100), such that the foot (162) of the user is positioned at a default position for using the function for adjusting the setting of the optical imaging system (100).

10. The apparatus (130) according to any one of the preceding claims, wherein the apparatus (130) is configured to
generate the adjustment data by determining, based on the movement data, gesture data indicative of a gesture performed by the user to control the optical imaging system (100) with the foot (162).

11. The apparatus (130) according to any one of the preceding claims, wherein the apparatus (130) is configured to:
determine, based on the movement data, an authorization of the user of the function for adjusting the setting of the optical imaging system (100); and
adjust a functionality of the function for adjusting the setting of the optical imaging system (100) based on the determined authorization.

12. An optical imaging system (100) (100), comprising
an apparatus (130) according to any one of the preceding claims.

13. The optical imaging system (100) according to claim 12, further comprising an illumination source configured to illuminate a part of the surrounding of the optical imaging system (100) with non-visible light.

14. A method (300) for an optical imaging system, comprising:
obtaining (310) sensor data indicative of a surrounding of the optical imaging system;
determining (320), based on the sensor data, movement data indicative of a movement of a foot of a user of the optical imaging system;
generating (330), based on the movement data, adjustment data for adjusting a setting of the optical imaging system; and
transmitting (340) the adjustment data for adjusting the setting of the optical imaging system.

15. A computer program with a program code for performing the method (300) according to claim 14 when the computer program is executed on a processor.
